(19) Europäisches Patentamt

European Patent Office

Office européen des brevets

(11) **EP 1 260 863 A1**

(12) **EUROPEAN PATENT APPLICATION**

(43) Date of publication:
**27.11.2002 Bulletin 2002/48**

(51) Int Cl.7: **G03F 7/00**, C12N 5/00

(21) Application number: **01610052.1**

(22) Date of filing: **23.05.2001**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI**<br><br>(71) Applicant: **Scandinavian Micro Biodevices**<br>**2800 Lyngby (DK)**<br><br>(72) Inventors:<br>• **Bouaidat, Salim**<br>**2200 Copenhagen N (DK)** | • **Winther-Jensen, Bjoern**<br>**2100 Copenhagen Oe (DK)**<br>• **Jonsmann, Jacques**<br>**3330 Gorlose (DK)**<br><br>(74) Representative: **Plougmann & Vingtoft A/S**<br>**Sundkrogsgade**<br>**P.O. Box 831**<br>**2100 Copenhagen O (DK)** |

(54) **Micropatterning of plasma polymerized coatings**

(57)  This invention relates to a lift-off process for micro-patterning of plasma-polymerised coatings using micro lithography. The plasma-polymerised surface may be hydrophobic, hydrophilic, cell non-adhesive surface (i.e. a surface which prevents bio-fouling), cell-adhesive surface (surface with high adhesion to cells thereby promoting cell-attachment), conducting, acidic, alkaline, etc. The invention enables patterning of a single or multiple plasma polymerised surfaces on the same substrate. The invention also relates to substrates and devices for use in cell sorting or cell-capture procedures.

**EP 1 260 863 A1**

## Description

FIELD OF THE INVENTION

This invention relates to micro-patterning of plasma-polymerised coatings using micro lithography. The plasma-polymerised surface may be hydrophobic, hydrophilic, cell non-adhesive surface (i.e. a surface which prevents bio-fouling), cell-adhesive surface (surface with high adhesion to cells thereby promoting cell-attachment), conducting, acidic, alkaline, etc. The invention enables patterning of a single or multiple plasma polymerised surfaces on the same substrate. The main advantage of this invention compared to other patterning techniques is the achievable feature size and the mentioned possibility of patterning multiple polymer surfaces with accurate alignment.

BACKGROUND OF THE INVENTION

The methods used today in cell-based drug screening are usually tedious and labour consuming. Electrical measurements are typically conducted on single cells under microscope. Here, microelectrodes are positioned on the individual cells manually using micro-manipulators. The cells are then exposed to different drugs, one at a time, and the potential or conductivity etc. is measured. In the case of optical measurements, for instance fluorescence, a cluster of cells (that produce fluorescent proteins) are placed in a flow cell. The drug is introduced to the cluster through a flow system, and the fluorescence they produce is detected using a fluorescence microscope. In today's High Throughput Screening (HTS), optical screening is usually conducted the following way. Cells are dispensed, using a dispenser robot, into an array of wells on a transparent microtiter plate. Next, a set or sets of drugs (drugs are thereby mixed on-site) is dispensed into the wells, and the measurements are performed Detectors are typically placed over the microtiter plate and a light source underneath. One advantages of the technique is the fact that it is highly automated and a number of drugs can be screened simultaneously. Disadvantages include the fact that larger number of cells and large volumes of drugs (typically from a couple of microliters) are needed in order to do the measurements. Also, the dimensions of the wells limit the number of measuring sites in the array.

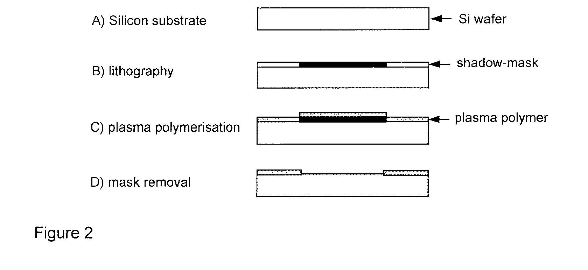

The prevailing method for patterning of plasma polymerised surfaces is shadow masking (see Figure 2). This is a very simple technique, where patterned metal film or cello tape is placed directly onto the substrate, see Figure 2B. The polymer is then deposited through the shadow-mask (Figure 2.C) and the mask is dissolved/etched (Figure 2D). The lowest achievable feature size of this technique is in the region of 20 μm - the limit is however closer to 50 μm for most practical purposes. Another disadvantage of shadow masking is, that is very difficult to pattern multiple surfaces, because accurate alignment is impossible. The poor feature size and inability to do accurate alignment makes the method unsuitable for the intended applications, since the smallest cell size is in the range of 1-2 μm.

Y. Pan *et al.*, A Precision Technology For Controlling Protein Adsorption And Cell Adhesion In Biomems. In *MEMS'01, The 14th IEEE International Conference on Micro Electro Mechanical Systems*, pages 435-438, 21-25 January 2001, Interlaken, Switzerland, describes a lift-off process for micropatterning in which plasma polymerised tetraglyme (pp4G) was deposited on silicon wafers. The polymerised tetraglyme provides cell non-adhesive surfaces on the surface.

C.H. Thomas *et al.* in Transactions of the ASME, Vol. 121, 1999, discloses a process for the preparation of a substrate on which single cells can be isolated on isolated patches of amino-terminated silane surrounded by a non-adhesive hydrogel of acrylamide and polyethyleneglycol.

US 5,470,739 discloses a process for providing a patterned design useful as a cell culture support. Patches of the surface are coated with collagen which influences cell-adhesion.

Although, certain methods exist for providing micro-structured devices, there is a need for further improved and more flexible methodologies for micropatterning in which strongly binding polymers and even polymers with different surface properties can be provided on the same substrate.

BRIEF DESCRIPTION OF THE DRAWINGS

Figure 1: Chips with arrays of measuring points. Left: An 8x8 array of small cell-adhesive surface patches where single cells can be fixated/positioned. A non-adhesive area surrounds the patches. Right: An 5x5 array of hydrophilic patches with small cell-adhesive surface patches surrounded by a hydrophobic area.

Figure 2: Process sequence for the shadow masking technique

Figure 3: Process sequences (standard lift-off) for micropatterning of one polymer surface. For multiple layers the sequence is simply repeated.

Figure 4: Process sequence for micropatterning of a polymer on a substrate. A metal (e.g., aluminium) is used as sacrificial layer in the lift-off).

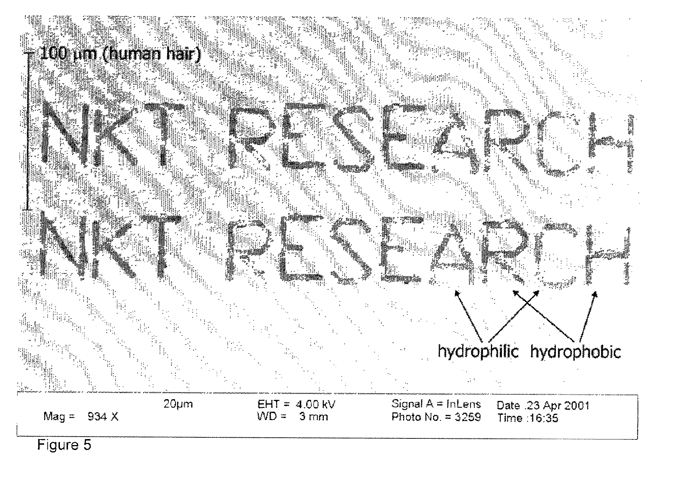

Figure 5: Scanning Electron Microscope (SEM) image of a silicon oxide surface with micro-patterned plasma polymers. The letters are "written" with alternate hydrophilic and hydrophobic letters. The letters have a height of approximately 35 $\mu$m and a width of 25 $\mu$m.

DESCRIPTION OF THE INVENTION

The present invention provides a lift-off process for micropatterning of a polymer on a substrate, said process comprising the steps of

(a) providing the substrate having a sacrificial layer in a predetermined micro-pattern;
(b) depositing a polymer layer on the sacrificial layer/substrate;
(c) dissolving/etching the underlying sacrificial layer (lift-off),

characterised in that the polymer layer is constituted by a cross-linked polymeric material prepared by plasma polymerisation of a monomer gas in a plasma, said monomer gas comprising one or more types of monomers selected from

(i) substituted benzenes, and
(ii) (halo)aliphatic compounds of the general formula $C_zH_yX_x$ wherein X is fluoro, chloro, bromo or iodo, z is 1-16 and x+y is 2z+2, 2z, 2z-2 or 2z-4.

The present invention also provides a substrate prepared according to the process.
The present invention furthermore provides a device having polymer patches and/or areas.

**Lift-off**

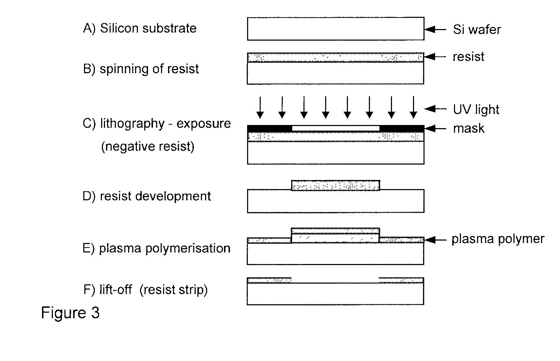

The process includes the step of providing a substrate with a sacrificial layer in a predetermined micro-pattern, that is a micro-pattern which is complementary to the desirable micro-pattern of the polymer.
The sacrificial layer is dissolved/etched after the polymer layer is deposited on the substrate. The polymer is normally deposited in such a manner that it covers the parts of the substrate which are not covered by the sacrificial layer as well as the parts of the substrate already covered by the sacrificial layer. When the sacrificial layer is dissolved/etched, a micro-pattern of the polymer which is complementary to the pattern of the sacrificial layer is revealed.
The term sacrificial layer is intended to cover conventional materials used in micropatterning of semiconductor devices by lift-off i.e. photoresists, metals, oxides, ceramics, polymers etc.
The fabrication of micro-patterned surfaces using micro lithography is based on the well-known lift-off technique (see, e.g., S. M. Sze, Semiconductor Devices, Physics and Technology, John Wiley & Sons, 1985, pp. 441-442). The process sequence is depicted on Figure 3 and will be presented by an illustrative example in the following where a negative resist is used.
A thin UV-sensitive photoresist, typically between 1.5-4.2 $\mu$m, is spun onto a standard Si wafer (Figure 3.B). After a short bake, typically 90°C for 30 sec. on a hotplate (depending on the resist thickness), the resist is exposed to UV-light through a mask. The pattern of the mask is thereby transferred to the resist (Figure 3.C). The unexposed resist is dissolved/etched (negative resist process) in a developer, see Figure 3.D. Plasma polymer is then deposited using the technique described further below (Figure 3.E). Finally, the underlying resist is dissolved/etched (thereby "lifting off' the undesired plasma polymer). The lift-off is performed using acetone in an ultrasonic bath (Figure 3.F). The process sequence is simply repeated for multiple polymer layers.
Thus, in one embodiment, the process comprises the steps of

(a) spinning a UV-sensitive photoresist on the substrate;
(b) masking the resist with a predetermined pattern and exposing the resist to UV light through the mask;
(c) developing the resist;
(d) depositing a polymer layer on the resist/substrate;
(e) dissolving the underlying UV-sensitive photoresist (lift-off).

The techniques can be modified as will be understood by the person skilled in the art with due considerations of the type of substrate and the desired alignment accuracy. It should also be understood that a positive resist can be used with the necessary modifications.

The micropatterning process of the invention has a lowest achievable feature size of approximately 1 μm and an alignment accuracy of approximately 2 μm, which is adequate for the diagnostic applications described further below.

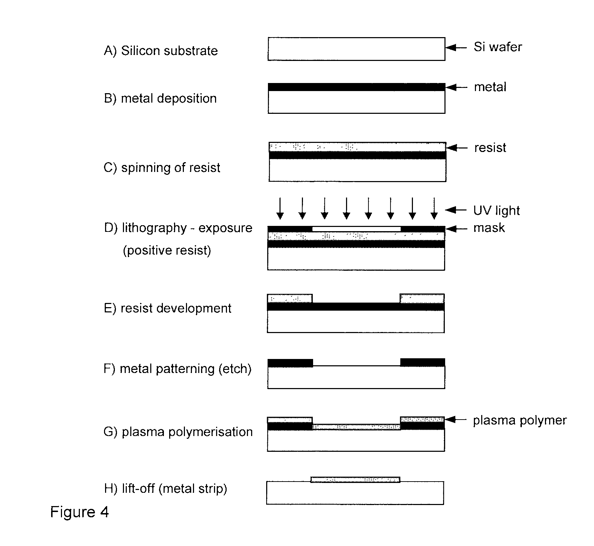

Another way of providing a temporary micro-pattern is to dispose a metal e.g. aluminium. The process sequence could be as follows. Aluminium is deposited on a silicon substrate by electron-beam evaporation or sputtering (Figure 4.B). The next steps (see Figure 4.C-E) are equivalent to steps B-D in the standard photoresist lift-off mentioned above. A thin UV-sensitive photoresist is spun onto the substrate (Figure 4.C). After a short bake, the resist is exposed (through the mask, see Figure 4.D), and developed (Figure 4.E). Next, the aluminium is etched, using the photoresist as mask and the photoresist is dissolved in acetone (Figure 4.F). Possible aluminium etchants include; phosphoric acid (with an 2:16:1 ratio of $H_2O:H_3PO_4:HNO_3$ at 50°C), acidic acid (with an 5:76:3:15 ratio of $H_2O:H_3PO_4:HNO_3:CH_3COOH$ at 40°C) or simply NaOH. The polymer is then plasma deposited by the technique described further below (Figure 4.G), and the lift-off, with aluminium as sacrificial layer, is performed in the same etchant as before (Figure 4.H).

**Plasma polymerisation**

The process of the present invention involves the step of deposition of a polymer layer which is constituted by a cross-linked polymeric material prepared by plasma polymerisation of a monomer gas on a surface, said monomer gas comprising one or more types of monomers selected from

> (i) substituted benzenes, and
> (ii) (halo)aliphatic compounds of the general formula $C_zH_yX_x$ wherein X is fluoro, chloro, bromo or iodo, z is 1-16 and x+y is 2z+2, 2z, 2z-2 or 2z-4.

When used herein, the term "polymeric material" is intended to mean a material prepared from a mixture of discrete low molecular weight compounds. Whether such low molecular weight compounds ("monomers") include groups which are considered as polymerisable in the normal sense, e.g. vinyl groups, is irrelevant for the purpose of the definition. Rather, the compounds used as "monomers" will, in particular in the presence of the substituted benzenes, form a highly cross-linked structure under the plasma polymerisation conditions in that the aromatic double bonds of the benzene system apparently undergo some kind of polymerisation via radical generation. The monomers will most often also form strong covalent bonds with the substrate, e.g. Si-C-R bonds with Si-H groups of a hydrogen-treated silicon substrate (Example 1). One very interesting observation is that number of double bonds originating from the benzene structure reduced dramatically or are virtually absent in the polymeric material. This observation has led the present inventor to believe that the method as well as the materials obtained by the method are uniquely novel.

As will be understood from the above, the term "monomer" is intended to mean a low molecular weight species. Most often, the monomers have a molecular weight of at the most 450. Preferred monomers are those having a molecular weight of at the most 200.

In one embodiment, the plasma polymerisation is effected with a monomer gas comprising one or more types of monomers selected from substituted benzenes. The substituted benzenes may - as a matter of definition - have 1-6 substituents, but will most often have 1-4 substituents, preferably 2-4 substituents, in particular 2-3 substituents.

It is believed that preferred substituted benzenes are those which have no polymerisable groups such as alkenyl, alkynyl, etc. substituents.

Examples of suitable substituted benzene monomers have the general formula

$$Ar(R^n)_n$$

wherein Ar is a benzene ring, n is 1-6 and $R^n$ is n substituents ($R^1, R^2, R^3, R^4, R^5, R^6$) covalently bound to the benzene ring, the substituents ($R^1, R^2, R^3, R^4, R^5, R^6$) being independently selected from $C_{1-6}$-alkyl, $C_{1-6}$-alkenyl, $C_{1-6}$-alkynyl, $C_{1-6}$-alkoxy, $C_{1-6}$-alkylcarbonyl, $C_{1-6}$-alkylcarbonyl, $C_{1-6}$-alkoxycarbonyl, carbamoyl, mono- and di($C_{1-6}$-alkyl)-aminocarbonyl, formyl, hydroxy, carboxy, carbamido, thiolo, nitro, cyano, nitro, amino, mono- and di($C_{1-6}$-alkyl)amino, and halogen (fluoro, chloro, iodo, bromo), wherein the $C_{1-6}$-alkyl, $C_{1-6}$-alkenyl, $C_{1-6}$-alkynyl and $C_{1-6}$-alkoxy groups in the above may be substituted with substituents, preferably 1-3 substituents, selected from hydroxy, $C_{1-6}$-alkoxy, carboxy, amino, mono- and di($C_{1-6}$-alkyl)amino and halogen.

In the formula above, it is preferred that the substituents are selected from $C_{1-2}$-alkyl, amino, and halogen.

Particular examples of suitable substituted benzenes are p-xylene, m-xylene, o-xylene, o-methylaniline, m-methylaniline, 2,3-dimethylaniline, 2,4-dimethylaniline, 2,5-dimethylaniline, 2,6-dimethylaniline, 3,5-dimethylaniline, fluorobenzene, etc.

Particularly useful substituted benzenes are those having two or more substituents where one substituent is a methyl group.

Also preferred are benzenes which do not include alkenyl or alkynyl substituents.

Preferred substituted benzenes are those having at least two substituents.

This being said, it is believed that particularly useful substituted benzenes are those which do not include oxygen atoms. This is particularly true when the substrate is silicon-containing in that any formation of Si-O-C bonds (which may be susceptible to hydrolysis with strong bases) between the oxygen atom of the substituted benzene and silicon atoms of the substrate is thereby avoided. Preferably less than 5 mol% of the monomers of the substituted benzene type comprises oxygen atoms.

As will be obvious from the above, the monomer gas may comprise other monomer types than the substituted benzene monomers. In order to fully benefit from the advantageous properties provided with the substituted benzene monomers, it is believed that the content of substituted benzene monomers, should be at least 5 mol%, although the content can be even higher such as in the range of 10-100 mol%, e.g. 25-100 mol%, such as 75-100 mol%, and often about 100%.

The amount defined for the substituted benzenes may encompass one or two or more different substituted benzenes, normally however, only one is used or two are used together.

As mentioned above, it has been found that the double bonds originating from the benzene structure often are virtually absent in the polymeric material prepared from the substituted benzenes. This being said, the materials will generally have less than 20% of the double bonds originating from the benzene structure of the substituted benzenes left. Typically as little as less than 10% of the double bond, preferably less than 5%, of the double bonds originating from the benzene structure of the substituted benzenes are left. This appears to be an important characteristic of the materials of the present invention.

The amount of double bonds left can be determined by FT-IR measurement or by reaction with bromine or fluoro compounds followed by elemental analysis with EPS (X-ray Photoelektron Spectroscopy).

The cross-linked polymer may also be provided with a (halo)aliphatic compound of the general formula $C_zH_yX_x$ wherein X is fluoro, chloro, bromo or iodo, z is 1-16. The number of substituents, i.e. the sum x+y, is 2z+2, 2z, 2z-2 or 2z-4 corresponding to straight chain (halo)aliphatic compounds, (halo)cycloaliphatic compound or monounsaturated (halo)aliphatic compounds, and diunsaturated (halo)aliphatic compounds or mono unsaturated (halo)cycloaliphatic compounds, etc. respectively.

The term "(halo)aliphatic compound" is intended to mean an aliphatic compound optionally being substituted one or more times with fluoro, chloro, bromo or iodo, preferably fluoro or chloro, such as perhalogenated compounds.

Examples of preferred (halo)aliphatic monomers for the process of the invention are:

a) Alkynes        $HC{\equiv}CH$, $HC{\equiv}C\text{-}(CH_2)_r\text{-}CH_3$ $(0{\leq}r{\leq}14)$.
b) Alkenes        $H_2C{=}CH_2$ and $H_2C{=}CH\text{-}(CH_2)_r\text{-}CH_3$ $(0{\leq}r{\leq}14)$ and cyclic alkenes.
c) Alkanes        $CH_4$, $CH_3\text{-}(CH_2)_r\text{-}CH_3$ $(0{\leq}r{\leq}14)$ and cyclic alkanes.
d) Haloalipatics        Monomers from group a, b and c containing one or more halogen atoms (F, Cl, Br and I).

The compounds can be utilised either in combination with the substituted benzenes or alone. The (halo) aliphatic compounds provides in themselves hydrophobic surface properties upon plasma polymerisation.

Examples of fully or partially halogenated organic compounds (haloaliphatic compounds) are perfluorocyclohexane, perfluorohexane, perfluoromethylpentene, difluroethylene, etc.

When the (halo)aliphatic compounds are used, it is believed that the content of (halo)aliphatic monomers should be at least 5 mol%, although the content can be even higher such as in the range of 10-100 mol%, e.g. 25-100 mol%, such as 75-100 mol%, and often about 100%. In one embodiment, the (halo)aliphatic compounds are used in combination with the substituted benzenes.

Other types of monomers can be used as the balance in the monomer gas in order to modify the properties of the materials. Such other types of monomer may be provided either by preparing a mixture of monomers to be applied simultaneous or by providing alternating amounts of different monomer types so as to form a virtual mixture in the plasma environment.

Illustrative examples of other monomer types to be selected from vinylacetate, vinylpyrolidone, ethyleneglycolvinylether, diethyleneglycolvinylether, methyl methacrylate, methyl methacrylate, allylalcohol, vinylbornene, acid anhydrides (in particular carboxylic acid anhydrides), acid halides (in particular carboxylic acid halides) such as acid chlorides, acid bromides, acid fluorides, acid iodides, epoxides, aldehydes, carboxylic acids, and thiols. In addition to the monomers above, the monomer gas may also comprise gaseous monomers such as $NH_3$, $N_2$, $N_2O$, $CO_2$, etc.

The plasma type advantageously used in the concept of the present invention is typically one generated by a multiple phase AC supply or a DC supply. It has been found that this type of plasma has a level of intensity which allows a substantial portion of the functional groups to be preserved. It is particularly advantageous to utilise a two or three phase AC plasma which offers the possibility of using a sufficiently low energy, e.g. energy levels of at the most 15 W/l such as at the most 10 W/l. Preferably, the intensity of the plasma is in the range of 10 mW/l to 15 W/l, such as 10 mW/l to 10 W/l, e.g. 10 mW/l to 5 W/l. Other types of generators of the plasma may also be applicable, e.g. RF and MF plasma and pulsed variants thereof.

The pressure in the reaction chamber will normally be in the range of 10-1000 µbar, such as 25-500 µbar. The pressure in the reaction chamber is controlled by a vacuum pump, and a supply of an inert gas and the monomer gas. The inert gas is suitably a noble gas such as helium, argon, neon, krypton or a mixture thereof.

Hence, a plasma reaction chamber can be adapted in accordance with the instructions given herein with possible modification obvious for the person skilled in the art.

The plasma polymerisation process is normally conducted for a period of 10-1000 sec, such as 20-500 sec.

The plasma polymerised material can be provided on the substrate in a substantially uniform thickness if desired. It is believed that the layer thickness of the material generally is in the range of 5-5000 nm, such as in the range of 10-1000 nm, typically 10-200 nm.

A wide range of substrates are suitable in the method of the invention, thus typically the solid substrate essentially consists of a material selected from polymers, e.g. polyolefins such as polyethylene (PE) and polypropylene (PP), polystyrene (PS), or other thermoplastics such as polytetrafluoroethylene (PTFE), tetrafluoroethylene-hexafluoropropylencopolymers (FEP), polyvinyl-difluoride (PVDF), polyamides (e.g. nylon-6.6 and nylon-11), and polyvinylchloride (PVC), rubbers e.g. silicone rubbers, glass, silicon, paper, carbon fibres, ceramics, metals, etc. Presently preferred materials are silicon, polyethylene (PE), polystyrene (PS) and glass, of which silicon is particularly interesting in view of the particularly useful products for micro-structuring lift-off techniques.

The substrate (and sacrificial layer) may be pre-coated or pre-treated in order to modify the properties thereof, e.g. the ability of the surface to adhere to the plasma polymerised material, or the hydrophobic or hydrophilic properties of the substrate as such. The pre-coating may (also) be performed by plasma polymerisation. In the case where the substrate is silicon, it is preferred that the surface therefor is pre-treated with a hydrogen plasma before the material is provided onto the silicon in that occasional Si-OH groups are thereby converted to Si-H groups. This step can be performed as a pre-step to the step of providing the polymer layer (see Example 1). Also interesting is pre-treatment with argon so as to clean and possibly activate the surface prior to plasma polymerisation with the substituted benzene.

The surface properties of the polymer is determined by the substituted benzenes and/or (halo)aliphatic compounds used in the plasma polymerisation process, as well as by any additional monomers used as the balance of the monomer gas.

Besides the fact that the polymer in itself may provide specific surface properties, it is also possible to modify the surface properties of the polymer even before the sacrificial layer is dissolved/etched.

As will be understood, hydrophobic, hydrophilic, cell-adhesive and cell non-adhesive surface properties of predefined patches (defined by the micro-pattern) for the substrate may be provided either by mixing suitable monomers with the substituted benzenes or may provided by plasma polymerisation of a further layer on top of the cross-linked material (plasma polymerised monomer gas comprising substituted benzenes). One advantage of the present process resides in the fact that the plasma-polymerised substituted benzenes provides a durable cross-linked material which has a strong bonding to the substrate (and the sacrificial layer). It is therefore possible to manipulate the substrate and to provide further layers without affecting the integrity of the pattern provided with the cross-linked material (and optional further layers on top thereof).

Thus, the surface properties can be provided by the monomer gas as such; by components provided by plasma polymerisation in succession of plasma polymerisation of the monomer gas; or by "wet" chemical modification of the polymer surface.

In one important embodiment, the polymer layer is functionalised prior to dissolution (or etching) of the underlying sacrificial layer.

The monomer gas as such can provide either a hydrophobic surface, i.e. when substituted benzenes with substituents other than amino, mono- and di($C_{1-6}$-alkyl)amino, thiolo and hydroxy are used and when the (halo)aliphatic compounds are used, or a hydrophilic surface, i.e. when substituted benzenes with amino, mono- and di($C_{1-6}$-alkyl) amino, thiolo and hydroxy substituents are used.

As it is illustrated in the examples, a further plasma polymerised layer may be provided on top of the material defined above. Such a layer may provide modify the surface properties of the polymer and may provide special surface properties to the material, e.g. hydrophobicity, hydrophilicity, cell-adhesive surface property, cell-repellence properties, etc.

The further plasma polymerised layer may be provided in direct succession of the polymer layer, i.e. as in illustrated in Example 2 where the monomer gas containing the substituted benzene is turned off after which the 1-vinyl-2-pyrolidone monomer is provided. This direct succession of monomers provides a very strong bonding between the two layers. Alternatively, the further plasma polymerised layer may be provided in a separate process.

In the process of preparing the cross-linked materials it is possible to add a monomer which provides hydrophilic properties either simultaneously with plasma polymerisation of substituted benzene or subsequent to plasma polymerisation of substituted benzene (see Example 2). Examples of monomers which can be combined with substituted benzenes or which can be polymerised in a subsequent step are acrylic acid, vinylacetate, ethanol, ethylenediamine, allylamine, vinylpyrolidone, vinylaniline, imidazoles such as vinylimidazole, or a mixture of acetylene and $N_2O$, etc.

When used herein, the term "cell non-adhesive surface" is intended to refer to a non-adhesive surfaces with regards to cells, i.e. a surface which prevents bio-fouling.

Examples of monomers which in combination with substituted benzenes or in a subsequent step provides a cell non-adhesive surface are polyethyleneglycols and derivatives, e.g. triethyleneglycol ("triglym") and diethyleneglycol-vinylether (DEGVE), as well as silicone monomers, e.g. hexamethylcyclotrisiloxane ($D_3$).

When used herein, the term "cell-adhesive surface" is intended to refer to a surface with predominant adhesion to cells, i.e. a surface which promotes cell-attachment.

Examples of monomers which provides a cell-adhesive surface area are acid and/or base groups, e.g. acrylic acid and allylamine, vinyldifluoride, and the like.

Thus in one embodiment, the invention provides a substrate with a hydrophilic surface.

In another embodiment, the invention provides a substrate with a hydrophobic surface.

In a further embodiment, the invention provides a substrate with a cell-adhesive surface.

In a still further embodiment, the invention provides a substrate with a cell non-adhesive surface.

In an even still further embodiment, the invention provides a substrate with a surface which is either (a) hydrophilic and cell-adhesive, (b) hydrophilic and cell non-adhesive, (c) hydrophobic and cell-adhesive, or (d) hydrophobic and cell non-adhesive. Thus, the substrate may have a surface which have more than one functionality. This, makes it possible to accurately design the surface to, e.g., discriminate between various types of cells.

The various surface properties can also be provided by "wet" chemical process. As an example, conducting surfaces may be provided in a subsequent step, i.e. after polymerisation of the substituted benzene (and other monomers), by means of combinations of monomers and constituents already known in the art, e.g. electropolymerisation of pyrrole (see, e.g., "Preparation and Characterisation of Processable Electroactive Materials, Anders Ravn Sørensen, Ph.D. Thesis, July 1993, ATV Erhvervsforskerprojekt EP 336, Denmark) and sputtering of metals, e.g. platinum, in thin (1-100 nm) layers, and subsequent electrochemical method for the preparation of thick (0.5-10 $\mu$m) metal layers, e.g. copper layers.

Furthermore, "wet" chemical modification of the surface of the plasma polymerised polymer may also involve reaction with any functional groups of the polymer, e.g. amino groups, etc. so as to attach entities with specific affinity for biomolecules, e.g. proteins, cells, DNA, RNA, PNA, LNA, etc.

It is particularly interesting to provide medical devices or bio-devices on which a plurality of patches or areas. Specifically, the present invention provides a device comprising a micro-patterned structure, said device comprising a substrate and a plurality of patches and/or areas comprising a cross-linked material prepared by plasma polymerisation of a monomer gas in a plasma, said monomer gas comprising one or more types of monomers selected from

(i) substituted benzenes, and
(ii) (halo)aliphatic compounds of the general formula $C_zH_yX_x$ wherein X is fluoro, chloro, bromo or iodo, z is 1-16 and x+y is 2z+2, 2z, 2z-2 or 2z-4.

In one embodiment, the process steps are repeated two, three or more times whereby patches having different surface properties can be provided on the same substrate. In particular, this makes it possible to provide a device, wherein the plurality of patches comprising the cross-linked material represent any of the following combinations of surface properties:

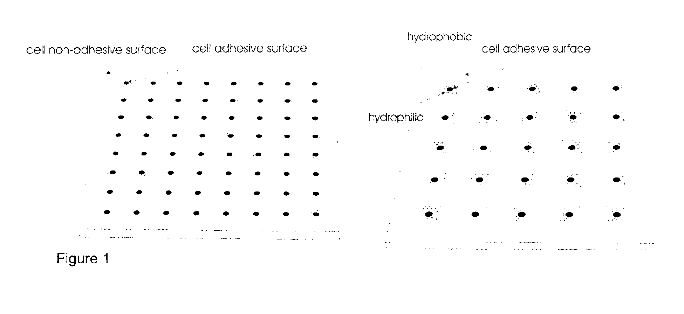

(i) a subset of the patches and/or areas having a cell non-adhesive surface and a subset of the patches and/or areas having a cell-adhesive surface (see Figure 1, left).
(ii) a subset of the patches and/or areas having a cell-adhesive surface and a subset of the patches and/or areas having a hydrophobic surface;
(iii) a subset of the patches and/or areas having a cell non-adhesive surface and a subset of the patches and/or areas having a hydrophobic surface;
(iv) a subset of the patches and/or areas having a cell-adhesive surface and a subset of the patches and/or areas having a hydrophilic surface (see Figure 1, right); or
(v) a subset of the patches and/or areas having a cell non-adhesive surface and a subset of the patches and/or areas having a hydrophilic surface.

**Industrial application**

The material has proven particularly useful for providing tough cross-linked layers for the present micropatterning processes where the lift-off technique is utilised. The lift-off technique requires that the materials used can resist treatment with strong solvents such as acetone, toluene, dichloroethylene, THF, etc. or strong alkaline conditions such as NaOH solutions at pH 14, etc. As will be evident from Example 4, the cross-linked materials provided with the plasma polymerisation of substituted benzenes are extremely useful for lift-off processes. For removal of aluminium can be used phosphoric acid (with an 2:16:1 ratio of $H_20:H_3PO_4:HNO_3$ at 50°C) or acidic acid (with an 5:76:3:15 ratio of $H_20:H_3PO_4:HNO_3:CH_3COOH$ at 40°C) or simply NaOH.

Some important applications of the invention are believed to be diagnostics or drug discovery. These applications often require some sort of manipulation, immobilisation or positioning of biological entities such as cells, or liquids such as regents and drugs.

In cell-based screening, it is very desirable to be able to automatically position cells in an array and apply different test liquids to the cells - possibly fully integrated in a biomicrochip. Figure 1 depicts two possible schemes - Left: an array with small cell-adhesive surface patches, typically below 5 μm in diameter, where the cells are attached. The cell-adhesive surface patches are surrounded by a cell non-adhesive surface area, which prevents cell-attachment. Cells usually have high affinity to almost all surfaces. By deposition a polymer with suitable surface properties, for instance polyethylene glycol (PEG) or plasma-polymerised tetraglyme (pp4G), this can be prevented effectively (Y. Pan *et al.*, 435-438, MEMS'2001). Figure 1 (Right) depicts a more elaborate scheme where test liquids are positioned on top of the cells. Small cell-adhesive surface patches are surrounded by small hydrophilic patches, which in turn are surrounded by a hydrophobic area. The liquids are applied onto the hydrophilic area using a standard micro dispenser. The surrounding highly hydrophobic area constrains the liquids and cells to the small measuring sites. Separate measurements, either electrical or optical can then be conducted. Fluorescence measurement is an especially interesting application, since it is widely used in cell-screening. Detectors could be placed over the measuring sites and a light source underneath. This however requires a transparent substrate such as glass instead of silicon.

In the case of electrical measurements, different electrodes, for instance AgCl, Au, Pt etc. and different electrode configurations may be used. Electrodes can be located at each measuring point or wherever on the substrate.

Thus, the present invention has the potential of making especially the tedious electrical measurement methods obsolete, since it is a more or less fully automated array, which can screen a large number of drugs simultaneously. The main advantages of the present invention compared to optical HTS is, that single cell measurements are possible and that much smaller drug volumes (nanoliter range) are needed. Also, since the area of each measuring point is much smaller than that of the conventional HTS, a much bigger array per area is possible. The measurement procedure is very similar to the one described further above. It could be as follows: Cell suspension is dispensed over the measuring points in the array using a micro-dispenser. The small cell-adhesive surface patches fixate single cells. The rest of the cells are sucked away through holes in the substrate or by flushing the surface. Next, a set or sets of drugs are dispensed over the measuring points and the measurement is conducted. When the measurement is finished, the drugs are removed and a set of new drugs may be dispensed and new measurements are performed.

Particular applications which can be envisaged for the products of the present invention are:

1. Conducting circuits for micro sensors, in particular for environments of harsh chemical exposure.

    a. Micro sensors for the chemical industry, organic synthesis and analytical purposes, e.g. pH measurements, measurement of ionic strength, and measurement of salt concentrations.
    b. Micro sensors for biotechnological purposes, including sensors for implantation in living organisms (such as humans), e.g. sensors for measurement of blood sugar in diabetic patients, sensors for measurement/registration of nerve pulses (e.g. in muscles, skin, brain, spinal marrow) and sensors for measurement of conductivity in micro flow systems.

2. Conducting circuits for micro actuators, in particular for environments of harsh chemical exposure, and for biotechnological purposes including sensors for implantation in living organisms (such as humans).

    a. Actuators for stimulation of nerves in living organisms e.g. in muscles, skin, brain, spinal marrow.
    b. Actuators for controlling mechanical units, e.g. dosage pumps, in living organisms and in micro flow systems.

3. Conducting structures for establishing voltage fields which render it possible to control movement and/or fixation of, e.g., biomolecules such as cells and proteins by utilising the inherent charge of such molecules in combination with the applied voltage in the conducting structure.

a. Conducting structures for micro electrophoresis

b. Conducting structures for movement and fixation of cells and other biomolecules in micro flow systems.

4. Conducting circuits for electronic components.

5. Optically active/non-optically active structures or structures where various parts have different optical properties.

EXAMPLES

**Example 1**

The process was conducted as illustrated in Figure 3 (steps A-F) under the following conditions. Precise alignment between two or more polymer surfaces is achieved using the lithography equipment and alignment marks formed on the silicon substrate. The process (except Step E) is conducted following conventional methodologies (see e.g. "Lithography in Experimental Environment" by Hovinen, Malinin and Lipsanen, Helsinki University of Technology, in Reports in Electron Physics 2000/21, Espoo 2000).

Step A

Standard 4" silicon wafers (Okmetic Oy) on which $SiO_2$ is grown.

| $SiO_2$ growth: | |
| --- | --- |
| Equipment | Tempress system |
| Process | Wet oxidation |
| Temperature | 1000 °C |
| Pressure ($H_2O$) | 760 Torr |
| Time | 10 min. |
| Oxide film thickness | 1000 Å |

Step B

| Spinning of resist: | | |
| --- | --- | --- |
| Equipment | SSI 150 dual track spinner | |
| Resist | AZ5214E (Shipley Company, L.L.C) | |
| Thickness | 4.2 μm | |
| Dispensed volume | approx. 4 ml | |
| Spin | | |
| | Speed | 700 rpm |
| | Time | 40 s. |
| Rest | | |
| | Time | 150 s. |
| Bake (hotplate) | | |
| | Temperature | 90 °C |
| | Time | 120 s. |

Step C

| Lithography | |
| --- | --- |
| Equipment | Karl Suss MA/BA6 aligner |

(continued)

| Lithography | |
|---|---|
| Process Settings | Positive resist |
|     Mode | Soft contact |
|     Alignment gap | 20 μm (Gap between the resist and mask during alignment for alignment between to polymer surfaces) |
| Exposure gap | 0 μm (soft contact between mask and resist during exposure) |
| Exposure time | 16 s |
| Lamp power | 300 W |
| Wavelength | 365 nm |

Step D

| Development of resist | |
|---|---|
| Developer | AZ 351B (Shipley Company, L.L.C) |
| Dilution ratio to water | 1:5 (The developer is basically NaOH at pH 14) |
| Development time | 2.30 min |
| Water rise | 5 min |

Step E

The "developed" 4" silicon wafers were placed in a 135 litre 2-phase AC-plasma chamber. The pressure in the chamber was lowered to 0.05 mbar and a flow of 20 sccm argon was led into the chamber. A plasma of 5 W/litre was started. After 60 seconds the flow of argon was lowered to 10 sccm and a flow of hydrogen (10 sccm) was started. After another 30 seconds the argon flow stopped. After 60 seconds with hydrogen plasma, the hydrogen flow was stopped, a flow of p-xylene vapour (20 sccm) and a flow of argon (10 sccm) was started. The power was lowered to 3 W/litre. After 60 seconds with plasma polymerisation of p-xylene, the p-xylene flow was stopped. The plasma was turned off, all flows were stopped and the pressure was raised to atmospheric pressure.

The surface is first treated with argon so as to clean and possibly activate the surface. Hydrogen is then provided with the aim of reducing any Si-OH groups on the surface to Si-H groups so that the subsequently added p-xylene were able to form Si-C-R bonds with the silicon substrate. Without the reduction with hydrogen, it is believed that base labile Si-O-C-R groups could have been formed.

Step F

| Lift-off | |
|---|---|
| Solvent | Acetone |
| Ultrasound is used to promote the lift-off | |

**Example 2**

The process sequence from Example 1 was repeated except that Step E was modified as follows: A flow of 1 -vinyl-2-pyrolidon (15 sccm) was started after the xylene flow was stopped. The power was lowered to 0.5 W/litre and the pressure was raised to 0.1 mbar. The plasma polymerisation of 1-vinyl-2-pyrolidon was continued for 120 seconds. The plasma was turned off, all flows were stopped and the pressure was raised to atmospheric pressure.

The final layer of poly(vinyl pyrolidone) provides a hydrophilic functionality.

**Example 3**

The process sequence was performed twice in sequence on the same silicon wafer substrate where a hydrophobic pattern and a hydrophilic pattern was prepared as described in Example 1 and Example 2, respectively. The pattern is shown in Figure 4 where the letters are "written" with alternate hydrophilic and hydrophobic letters. The letters have a height of 35 μm and a width of 25 μm.

**Example 4**

Test coatings of plasma polymerised p-xylene on glass were refluxed in acetone and toluene, respectively, for 24 hours. The contact angle with water was subsequently measured in order to determine whether the coating was left on the substrate. All samples showed a contact angle of between 85° and 95° (water on glass would give a contact angle of approx. 40°). The measured contact angle was substantially equal to the contact angle measured before the refluxing.

Similar samples were tested in alkaline environment in solutions of NaOH. The coatings resisted a solution with pH 12.5 for more than 24 hours and a solution with pH 14 for more than 1 hour. It was therefore concluded that the coating layers were able to resist even harsh conditions as those prevailing in micropatterning lift-off processes.

**Example 5**

The process sequence was repeated as in Example 2 except that glass was used a substrate and diethyleneglycol-vinylether (DEGVE) was used instead of 1-vinyl-2-pyrolidone. The surface provided gave a contact angle of 10° and had cell non-adhesive properties.

**Claims**

**1.** A lift-off process for micropatterning of a polymer on a substrate, said process comprising the steps of

> (a) providing the substrate having a sacrificial layer in a predetermined micro-pattern;
> (b) depositing a polymer layer on the sacrificial layer/substrate;
> (c) dissolving/etching the underlying sacrificial layer (lift-off),

> **characterised in that** the polymer layer is constituted by a cross-linked polymeric material prepared by plasma polymerisation of a monomer gas in a plasma, said monomer gas comprising one or more types of monomers selected from

> (i) substituted benzenes, and
> (ii) (halo)aliphatic compounds of the general formula $C_zH_yX_x$ wherein X is fluoro, chloro, bromo or iodo, z is 1-16 and x+y is 2z+2, 2z, 2z-2 or 2z-4.

**2.** A process according to any of the preceding claims, wherein the polymer layer is functionalised prior to dissolution of the underlying layer.

**3.** A process according to any of the preceding claims, said process comprising the steps of

> (a) spinning a UV-sensitive photoresist on the substrate;
> (b) masking the resist with a predetermined pattern and exposing the resist to UV light through the mask;
> (c) developing the resist;
> (d) depositing a polymer layer on the resist/substrate;
> (e) dissolving the underlying UV-sensitive photoresist (lift-off).

**4.** A process according to any of the preceding claims, wherein the one or more types of monomers are selected from substituted benzenes.

**5.** A process according to claim 4, wherein the substituted benzene has the general formula:

$$Ar(R^n)_n$$

wherein Ar is a benzene ring, n is 1-6 and $R^n$ is n substituents ($R^1, R^2, R^3, R^4, R^5, R^6$) covalently bound to the benzene ring, the substituents ($R^1, R^2, R^3, R^4, R^5, R^6$) being independently selected from $C_{1-6}$-alkyl, $C_{1-6}$-alkenyl, $C_{1-6}$-alkynyl, $C_{1-6}$-alkoxy, $C_{1-6}$-alkylcarbonyl, $C_{1-6}$-alkylcarbonyl, $C_{1-6}$-alkoxycarbonyl, carbamoyl, mono- and di ($C_{1-6}$-alkyl)-aminocarbonyl, formyl, hydroxy, carboxy, carbamido, thiolo, nitro, cyano, nitro, amino, mono- and di ($C_{1-6}$-alkyl)amino, and halogen (fluoro, chloro, iodo, bromo), wherein the $C_{1-6}$-alkyl, $C_{1-6}$-alkenyl, $C_{1-6}$-alkynyl and $C_{1-6}$-alkoxy groups in the above may be substituted with substituents selected from hydroxy, $C_{1-6}$-alkoxy, carboxy, amino, mono- and di($C_{1-6}$-alkyl)amino and halogen.

**6.** A process according to claims 4 or 5, wherein less than 10% of the double bonds originating from the substituted benzene are left in the material.

**7.** A process according to any of the preceding claims, wherein the substituted benzene monomer or monomers constitute(s) at least 5% of the monomer gas.

**8.** A process according to any of the preceding claims, wherein the process steps are repeated two or more times.

**9.** A substrate prepared according to the process of any of the preceding claims.

**10.** A device comprising a micro-patterned structure, said device comprising a substrate and a plurality of patches and/or areas comprising a cross-linked material prepared by plasma polymerisation of a monomer gas in a plasma, said monomer gas comprising one or more types of monomers selected from

    (i) substituted benzenes, and
    (ii) (halo)aliphatic compounds of the general formula $C_zH_yX_x$ wherein X is fluoro, chloro, bromo or iodo, z is 1-16 and x+y is 2z+2, 2z, 2z-2 or 2z-4.

**11.** A device according to claim 10, wherein the plurality of patches comprising the cross-linked material represent any of the following combinations of surface properties:

    (i) a subset of the patches and/or areas having a cell-adhesive surface and a subset of the patches and/or areas having a hydrophobic surface;
    (ii) a subset of the patches and/or areas having a cell non-adhesive surface and a subset of the patches and/or areas having a hydrophobic surface;
    (iii) a subset of the patches and/or areas having a cell-adhesive surface and a subset of the patches and/or areas having a hydrophilic surface; or
    (iv) a subset of the patches and/or areas having a cell- non-adhesive surface and a subset of the patches and/or areas having a hydrophilic surface.

Figure 1

Figure 2

A) Silicon substrate ← Si wafer

B) spinning of resist ← resist

C) lithography - exposure ← UV light
(negative resist) ← mask

D) resist development

E) plasma polymerisation ← plasma polymer

F) lift-off (resist strip)

Figure 3

A) Silicon substrate — Si wafer

B) metal deposition — metal

C) spinning of resist — resist

D) lithography - exposure (positive resist) — UV light — mask

E) resist development

F) metal patterning (etch)

G) plasma polymerisation — plasma polymer

H) lift-off (metal strip)

Figure 4

Figure 5

**European Patent Office**

## EUROPEAN SEARCH REPORT

Application Number

EP 01 61 0052

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 4 371 407 A (KUROSAWA KEI) 1 February 1983 (1983-02-01) * column 3, line 38 - column 4, line 55 * --- | 1-3,8-10 | G03F7/00 C12N5/00 |
| X | DD 277 466 A (KARL MARX STADT TECH HOCHSCHUL) 4 April 1990 (1990-04-04) * the whole document * --- | 1,2,4-10 | |
| X | PATENT ABSTRACTS OF JAPAN vol. 013, no. 437 (E-826), 29 September 1989 (1989-09-29) & JP 01 165119 A (MATSUSHITA ELECTRIC IND CO LTD), 29 June 1989 (1989-06-29) * abstract * --- | 10 | |
| X | GOSAVI S ET AL: "Plasma-polymerized chlorinated alpha -methylstyrene (PP-C-alpha MS): a high performance negative electron resist" JAPANESE JOURNAL OF APPLIED PHYSICS, PART 1 (REGULAR PAPERS & SHORT NOTES), FEB. 1995, JAPAN, vol. 34, no. 2A, pages 630-635, XP002183921 ISSN: 0021-4922 * the whole document * --- | 10 | **TECHNICAL FIELDS SEARCHED (Int.Cl.7)** G03F C12N |
| D,A | US 5 470 739 A (AKAIKE TOSHIHIRO ET AL) 28 November 1995 (1995-11-28) --- -/-- | | |

The present search report has been drawn up for all claims

| Place of search | Date of completion of the search | Examiner |
|---|---|---|
| THE HAGUE | 23 November 2001 | Haenisch, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

## EUROPEAN SEARCH REPORT

Application Number

EP 01 61 0052

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | PAN Y V ET AL: "A precision technology for controlling protein adsorption and cell adhesion in bioMEMS" , TECHNICAL DIGEST. MEMS 2001. 14TH IEEE INTERNATIONAL CONFERENCE ON MICRO ELECTRO MECHANICAL SYSTEMS (CAT. NO.01CH37090) , 2001, PISCATAWAY, NJ, USA, IEEE, USA, PAGE(S) 435 - 438 XP002183922 ISBN: 0-7803-5998-4 | | |

**TECHNICAL FIELDS SEARCHED (Int.Cl.7)**

The present search report has been drawn up for all claims

| Place of search | Date of completion of the search | Examiner |
|---|---|---|
| THE HAGUE | 23 November 2001 | Haenisch, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 01 61 0052

This annex lists the patent family members relating to the patent documents cited in the above–mentioned European search report.
The members are as contained in the European Patent Office EDP file on
The European Patent Office is in no way liable for these particulars which are merely given for the purpose of information.

23-11-2001

| Patent document cited in search report | | Publication date | Patent family member(s) | | Publication date |
|---|---|---|---|---|---|
| US 4371407 | A | 01-02-1983 | JP | 57172739 A | 23-10-1982 |
| | | | JP | 1653253 C | 30-03-1992 |
| | | | JP | 3011090 B | 15-02-1991 |
| | | | JP | 57075440 A | 12-05-1982 |
| | | | DE | 3173581 D1 | 06-03-1986 |
| | | | EP | 0050973 A1 | 05-05-1982 |
| DD 277466 | A | 04-04-1990 | DD | 277466 A1 | 04-04-1990 |
| JP 01165119 | A | 29-06-1989 | NONE | | |
| US 5470739 | A | 28-11-1995 | JP | 5176753 A | 20-07-1993 |

EPO FORM P0459

For more details about this annex : see Official Journal of the European Patent Office, No. 12/82